# EUROPEAN PATENT APPLICATION

(11) **EP 1 876 242 A2**
(43) Date of publication of application: **09.01.2008**
(21) Application number: 07005186.7
(22) Date of filing: 26.02.2001
(51) Int. Cl.: C12N 15/82

(54) **A process for generating genetically modified pearl millet through agrobacterium and biolistic transformation**

(30) Priority: 25.02.2000 IN MA01552000
(62) Divisional of application: 01928188.0
(71) Applicant: Avestha Gengraine Technologies Pvt. Ltd, Bangalore 560 066, Karnataka (IN)
(72) Inventor: Patell, Villoo Morawala, Andhra Pradesh (IN); Krishnan, Ranjini, Kerala (IN); Chandran, Divya, Kanartaka (IN); Rayapuram, Naganand, Raichur 584 101 Karnataka (IN)
(74) Representative: Bates, Philip Ian

(57) **Abstract**

The present invention relates to a process for biolistic transformation and regeneration of *Pennisetum glaucum* (Pearl millet) comprising: a) initiating embryogenic calli formation from the seeds of *P. glaucam* in an MS media containing 5mg/L of 2,4 D; b) incubating the said calli in dark for a predetermined period; c) sub-culturing the calli on an MS media containing 3mg/L of 2,4 D; d) incubating said sub-cultured calli under light for a predetermined period; e) subjecting the embryogenic calli to biolistic bombardment with plasmid DNA containing pre-identified genes using a biolistic apparatus; f) allowing the proliferating calli to grow and differentiate into plantlets; g) analysing the expression of said pre-identified genes in the regenerated plantlets using known techniques.

## Description

### Field of invention

The present invention relates to a process for biolistic transformation and regeneration of Pearl millet, in particular transformation of pearl millet variety 843B which confers stress tolerance.

### Background

A wide range of environmental stresses (chilling, ozone, high light, drought, etc.) can damage crop plants, resulting in high yield losses. A common event during all these unrelated adverse conditions, called oxidative stress, is the enhanced production of activated oxygen species (AOS), are highly toxic to living cells are produced as byproducts in many biological reactions. It has been suggested that active oxygen species are involved in the damage to the plant cell that is caused by environmental stress such as air pollution, high temperature, low water content etc. During stress conditions generation of oxygen radicals are very high, these highly active oxygen radicals(O*) are involved in the following damages to the plant cell:
- Degradation of lipids
- Denaturation of proteins
- Breakdown of Nucleic Acids.

Chloroplast are potentially the major source of toxic oxygen derivatives in plant tissues. They generate singlet oxygen under high doses of illumination or any other stress conditions. Accumulation of active oxygen species is an unavoidable consequence of photosynthesis, even under the most favourable conditions. To cope with their toxicity, plants have developed a highly efficient anti-oxidant defense system, composed of both enzymatic and non-enzymatic constituents. In plants a number of enzymes involved in free radical scavenging are normally induced in response to a variety of oxidative challenges. However during prolonged stress conditions damage becomes inevitable because the detoxification system becomes saturated. The main players are superoxide dismutase(SOD), ascorbate peroxidases(APX) and catalases. By enhancing the levels of these proteins in transgenic plants, it was attempted to improve the tolerance against oxidative stress.

The chloroplast being one of the primary sites of AOS production requires enhanced protection and MnSOD targeted to the chloroplast is for efficient scavenging of AOS in the chloroplast. In eukaryotes. MnSOD is a nuclear-encoded protein, that scavenges superoxide radicals in the mitochondrial matrix. Superoxide radicals are ubiquitously generated in many biological oxidations within all compartments of the cell. Increased production of superoxide radicals is associated with a number of physiological disorders in plants. By targeting MnSOD enzyme to the chloroplast, where the generation of superoxide radicals is high during stress conditions, the capacity to scavenge any radical that may be produced can be increased. The chloroplast transit peptide of the small subunit of Rubisco (ssTP) had been used successfully used in both monocotyledonous and dicotyledonous plants to target different transgenic non-plastid proteins(Cashmore et al 1983).

This has been proved that dramatic induction of MnSOD during the stress conditions. It also has been suggested that superoxide radicals trigger a specific molecule in each subcellular compartment, which is capable of acting as a signal to induce nuclear gene encoding for the particular superoxide dismutase associated with that compartment (Bowler C et al., 1989).

The object of the present invention to enhance the levels of these proteins in transgenic plants and improving the tolerance against oxidative stress.

To achieve the said object the present invention provides a process for biolistic transformation and regeneration of *Pennisetum glaucum* (Pearl millet) comprising:
(a) initiating embryogenic calli formation from the seeds of *P. glaucam* in an MS media with 5mg/L of 2,4 D
(b) incubating the said calli in dark for a predetermined period
(c) sub-culturing the calli on an MS media 3mg/L of 2,4 D,
(d) incubated said sub cultured calli under light for a predetermined period
(e) subjecting the embryogenic calli to biolistic bombardment with plasmid DNA containing pre-identified genes using a biolistic apparatus,
(f) allowing the proliferating calli to grow and differentiate into plantlets
(g) analysing the expression of the pre-identified genes in the regenerated plantlets using known techniques

The variety of said *P. glaucam* used is *P.glaucam var. 843B.* Said pre-identified gene is a marker gene selected from GUS encoding gene, Hygromycin resistance gene or MnSOD gene.
The plasmids used in step (e) for transforming genes are selected from and pGV4 construct..Said pIG121Hm plasmid is used for conferring GUS and hygromycin resistance gene and contains CaMV35s promoter. Said pGV4 plasmid is used in step (e) is for providing MnSOD gene and contains ubiquitin promoter and NOS terminator.

The media used for differentiating the said calli obtained in step (f) into shoots, is MS basal medium with 0.3ppm on Benzyl Ammo purine (BAP).

The said calli was kept under light for a period of 30 days for shoot formation.

The media used for differentiating the said calli obtained in step (f) into root, is MS media with 2-3 ppm, Indole Acetic Acid (IAA) or 0.3 % of activated charcoal.

Said process further comprises acclimatizing the plant in sterile water for at least 2 days and transferring it into sterile soil. The sterile soil contains soil and sand in the ratio 1:1.
The calli is incubated under dark conditions in step (b) for 30 days. The calli is incubated under light conditions in step (d) for 30 days.

Said biolistic gun used for transformation is the PDS-1000/He gun. Said biolistic gun uses high velocity microcarriers selected from gold. The microcarriers are coated with DNA molecule, spermicide and CaCl₂ The concentration of said DNA molecule is about 5µg/µl. The size of gold used is between 1.5 -3.0 µ.

The rupture disc pressure is 900 psi, the helium pressure is 1100psi and a vacuum of 25mg/Hg was created in the gun chamber.

The calli was bombarded at step (c) at 6cms distance under rupture disc pressure of 900 psi and helium pressure of 1100psi showed best results.

### DETAILED DESCRIPTION OF THE INVENTION

### Step 1.: Collection of plant material

Seeds of Pearl millet variety 843B also called the ICMB -2 line is an inbred highly uniform line and is a selection of BKM2068 that was bred at Fort Hays branch experiment station, Kansas State University. It is a maintainer line of the male sterile line 843A . These were recommended by AICPMIP in 1984 for large-scale distribution and production of commercial hybrids. 843B is a d2 dwarf, is early in maturity (42 days), shows good tillering (4 -5panicles), has small semi compact panicles and large seeds (ICRISAT, millet research group,Hyderabad).

The seeds were rinsed in water and then treated with 70% ethanol for surface sterilization. They were then washed thoroughly with distilled water and sterilized with 50% commercial bleach solution for 20 minutes. The use of 0.1 % mercuric chloride was found to be useless as the seeds due to over exposure to these chemicals failed to callus. The seeds were given 6 thorough washes in the laminar airflow unit with sterile distilled water. The scutellar tissue in the explant proliferates in vitro and has the maximum potential for regeneration and organogenesis.

### Step 2:

Embryogenic calli were initiated from the seeds in MS media containing 5mg/l 2,4-D (MS5). These callus cultures were placed in the BOD at 25°C in the dark. The calli were shifted to fresh media every 5 days to reduce the browning in the calli caused due to the high phenolic content. The calli were subcultured at 30 days intervals into media containing decreasing concentrations of 2,4-D. The calli in the MS3 stage were used for biolistic transformation. The sterile seeds were placed in the MS5 medium at 30 seeds per plate count. The seeds start callusing within 2-3 days. The hard compact mass of embryogenic callus is found enclosed within a mass of soft calli which lack organisation. The embryogenic calli are subcultured into MS3 plates and are placed under light for a period of 30 days. It is at this stage that the calli are best suited for bombardment. The proliferating calli are passed onto MS 1 and later plain MS media for further differentiation. The calli in a shooting medium containing 0.3% BAP start proliferating very well and differentiate to form green shoots. These plates are kept under light. The plantlets are shifted to a rooting medium containing activated charcoal till fine white roots are formed. The mature plantlets are let to harden in sterile water for a couple of days before being transferred into pots containing 1:1 soil and sand mixture.

### Method used for Biolistic bombardment of embryogenic calli of pearl millet

- Two plasmids were used for separate sets of experiments. 1). pIG121Hm (16.2 kb) which contains the GUS coding sequence and the Hph gene which confers resistance to hygromycin under the control of CaMV35s promoter and the NOS terminator.2). pGV4 (4.2 kb) carrying the MnSOD sequence driven by the UBI promoter and the NOS terminator. Ubiquitin driven promoters are relatively stronger and more active than the other monocot promoters. It also aids in maintaining the stability of the transgene in the plant. (9).

### Biolistic apparatus and bombardment:

The biolistic gun used is the PDS-1000/He gun from Biorad . It is a device used to introduce foreign DNA into calli or other tissue with the help of high velocity microcarriers like gold or tungsten. Gold is used in our experiments because of its inert and non-reactive nature in the plant system. Helium gas is used to accelerate the microcarrier, which have been coated with DNA molecules. By this method any type of tissue can be used for transformation and false positive results are reduced to a great extent. Transient assay can be conducted easily. During standardization experiments different distances were tried for the calli plate from the stopping screens. Also three different stages namely MS5, MS3, MS1 of the calli were tested for the bombardment. The size of gold used was between 1.5-3.0µ. The rupture disc pressure was 900 psi while the helium pressure had to be 1100psi. A vacuum of 25mg/Hg was created in the gun chamber. The concentration of the DNA used was around 5µg/µl.

### The gold suspension:

6mg of gold particles were weighed to which 100µl of 100% ethanol was added and was vortexed for a minute. This was centrifuged for 10 seconds at 10000rpm. The supernatant was pipetted out and 100µl of sterile distilled water was added to the pellet. It was vortexed and centrifuged and the same procedure was repeated. 50µl final gold suspension was used for bombardment.

### Particle coating protocol:

To 50µl of the gold suspension 5µg of DNA was added and mixed well. To this 20µl of 0.1M Spermidine (Sigma Aldrich) was added and mixed at low speed on the vortex. 50µl of 2.5M CaCl₂ was added and mixed well. The mix was left at room temperature for 10 minutes. It was centrifuged for 10 seconds at 10000rpm and the supernatant was pipetted out and 50µl of 100% ethanol was added to the pellet.

### Bombardment:

The microcarrier with the bound DNA was coated on to a macrocarrier disc. After the ethanol evaporated the macrocarrier was placed in its holder. The rupture disc and stopping screens were put in place and the other necessary parameters also maintained. The calli were placed in osmoticum 4hrs prior to bombardment. Osmoticum had 30g/l of either Mannitol or Sorbitol. This step in the preparation of material for bombardment helps in increasing the transient expression of the bombarded genes. Transient expression and stable transformation of cells is facilitated through plasmolysis of the target cells. (10). Plasmolysed cells are less likely to extrude their protoplasm following penetration of cells by particles (10). After bombardment the calli were placed in osmoticum for 16 hrs in the dark. The calli were shifted to MS3 plates with 30mg/l hygromycin (1^{st} selection). For transient Gus assays the calli were transferred into Gus buffer overnight for 24hrs in the dark. The calli in the first selection medium were transferred into the second selection medium after 15 days (MS3with 50mg/l hyg). This was subcultured every 2 weeks until resistant colonies of calli were seen in the plates.

### G US assay

GUS activity was determined with x-Gluc as substrate in 0.2M phosphate buffer, triton-x 100%, chloramphenicol (100mg ml),/ sodium azide(100mg ml), milliQ water. The calli were incubated at37°c for 24hrs. (Jefferson's protocol)

### PCR analysis:

DNA was extracted for the purpose of PCR using the protocol of K.Edwards, Johnstone and Thompson (8). The calli were collected in a sterile eppendorf tube and macerated without the extraction buffer at room temperature for 15 minutes. 400µl of extraction buffer (200mM tris HCl, 250mMNaCl, 25mM EDTA, 0.5%SDS pH 7.5) was added and vortexed for 5 seconds. The extract was centrifuged at 13000rpm for 1 minute. 300µl of the supernatant was taken in a fresh tube and 300µl of iso-propanol was added. This was left at room temperature for 2 minutes. This was centrifuged at 13000 rpm and the pellet was vacuum dried for 5 minutes. To the dry pellet suitable amount of TE was added and the DNA stored at 4°C. The calli were taken after 30 days of selection in the first selection medium for the first screening. DNA was extracted from the calli using the above-mentioned protocol. The concentration of the DNA was found to be around 50ng/ µl. GUS fragment was used as the positive control. MnSOD fragment was used as the positive control for the other set of experiments. DNA from un-transformed callus of 843B was used as the negative control. The PCR analysis was performed in a 25µl volume of 2.5µl of PCR buffer, 1 ul of 50mM MgCl2, 0.5µl of 10mM dNTPs 0.5µl of forward primer and 0.5µl of reverse primer, 0.2ul of TaqDNA polymerase and 25ng of template DNA.GUS forward primer: 5"CCATACCTGTTCACCGACGA3", GUS reverse primer:
5"GGAATTGATCAGCGTTGGTG 3". SOD forward primer:
5"CTACGTCGCCAACTACAACAA3"; SOD reverse primer:
TAGTCTGGTCTGACATTCTTG 3" were used for the PCR analysis. PCR was performed using the Peltier thermal cycler (MJ) for 40 cycles of initial 3 minutes of 93°C, 45 secs of 93°C, 45 seconds of 50° and a final extension temperature of 72°C for 5 minutes. Amplified products were separated by agarose gel electrophoresis and stained with ethidium bromide.

### Southern blot analysis of the PCR products:

The PCR gel was used for the purpose of southern blotting. The gel was blotted for three hours and the DNA was transferred onto a nylon membrane (hybond-N, amersham). Prehybridisation was performed at 60°C for three hours (5% Dextran sulphate , 5x SSC, 0.1%SDS) with 2% w/v of liquid block. The membrane was probed using the GUS and MnSOD specific probes for 16 hrs. The membranes were washed twice with 1x SSC and 0.1% SDS at 60°C for 15 mins each. The membranes were incubated in a solution of buffer-A and liquid block for 1 hr at room temperature. The membranes were then treated with the antifluoroscein-AP conjugate antibody for 1 hour. The membranes were washed thoroughly with bufferA and 0.3% tween-20 twice for 5 mins each. They were placed with their DNA side on the CDP-star detection reagent in the dark. The excess reagent was wiped out and the plastic bag sealed before being exposed for 1hour and 24 hours to detect the desired bands. The probes used were the 2.08 kb Sac1 and BamH1 fragment of GUS from PAHC27 plasmid and 900bp pstl fragment of MnSOD fragment from pGV4 plasmid.

### Results:

### Plant regeneration and Bombardment

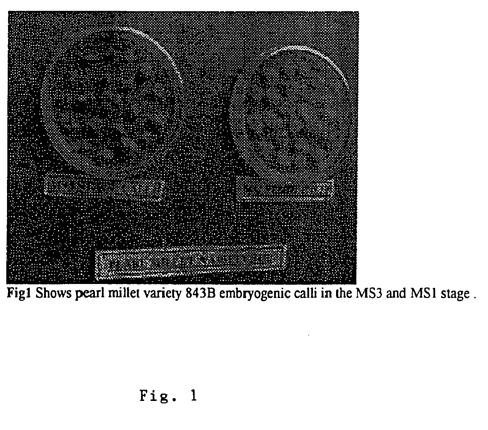

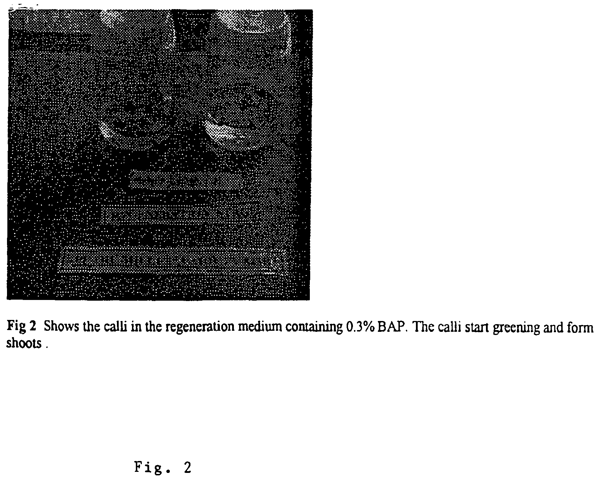

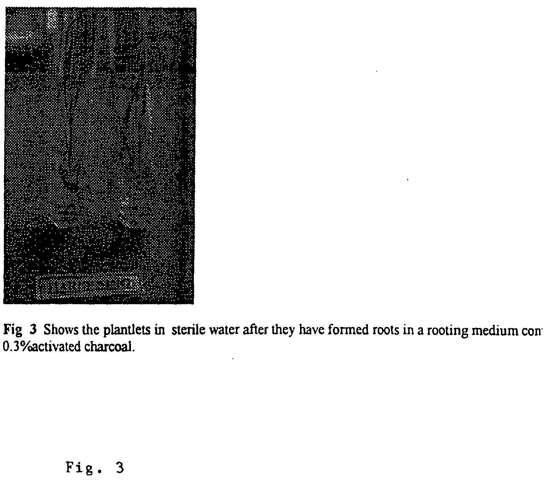

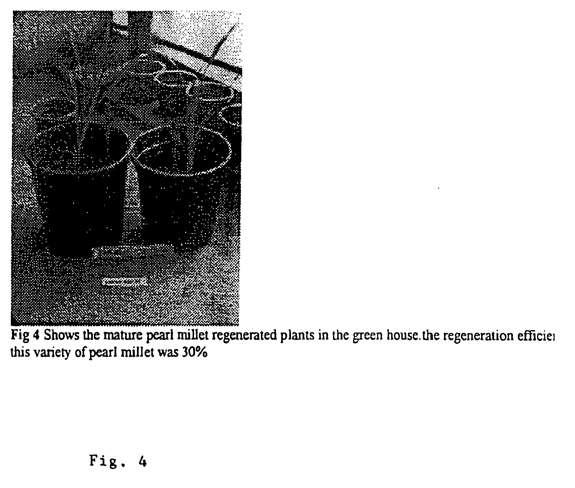

The scutellar region in cereal crops is a highly proliferating region, which can callus profusely giving rise to totipotent callus tissues. When pearl millet variety 843B, mature seeds were placed with their scutellar portion facing the medium the callusing rate was better than when they were placed with their scutellar regions away from the media. The callusing rate was very efficient in MS medium having 30g/l sucrose 10mg/l myoinositol and varying concentrations of 2,4-D (5mg/l, 3mg/l, and 1mg/l). light had a major effect on the callusing efficiency of the seeds. When MS3 plates were kept in the dark, the calli as is the case with all the cereals start producing phenolics to a greater extent than in the light. Hence the calli brown and the proliferation stops or gets retarded to a large extent. In order to prevent the browning phenomena, the calli were shifted into fresh MS media every 5 days and at the MS3 stage on wards they were placed in the light. One of the major drawbacks in millet tissue culture is the formation of spongy, soft white calli. These rarely or never undergo organization. These calli have to be removed occasionally to obtain the hard compact mass of embryogenic callus. The regeneration rate was 30% for *P.glaucum* var. 843B. The plants that were obtained by somatic embryogenesis have normal chromosome numbers and show the normal seed set. (Fig 1 to fig 4)

Three stages of callusing were used for bombardment. Of these stages the MS3 stage was found to be the most suited one. A distance of 6cms of the calli plate from the stopping screen was the ideal one for efficient transformation. This was proved by the transient Gus assays. Clear blue spots were seen in the calli confirming the presence of Gus gene in them.

### Selection of transformed calli

Embryogenic calli were bombarded with the GUS and MnSOD constructs. These were plated on to hygromycin containing selective media. A total of 20 resistant calli were obtained in a period of one month. No calli survived in the control plates. Growth of calli in the selective medium was similar to growth of calli in the non - transformed lines on non-selective medium. All the calli were resistant to hygromycin concentrations upto 50mg/l.

### PCR analysis:

PCR analysis of the randomly selected resistant calli was carried out using the appropriate primers for GUS and MnSOD. All the appropriate parameters were maintained during the runs. (Materials and methods). Of the 12 calli analysed for the presence of the GUS gene, 3 calli showed bands corresponding to the GUS gene in the positive control (2.0kb) (fig5). The transformation efficiency based on the PCR results was found to be 23%. The PCR was repeated to confirm the results. The PCR analysis of MnSOD bombarded calli showed one calli out of the 14 analysed having the desired MnSOD gene insert corresponding to the band in the positive control (900bp). In both the cases the negative control did not show any bands. The PCR was repeated to confirm the results.

### Southern blot analysis

The PCR gels were blotted for southern analysis for the presence of the GUS and MnSOD genes. On processing the blots, no signal was seen in the negative control lane. But in the case of MnSOD blot there was a faint signal in the negative control lane. This could be due to the presence of native MnSOD gene in Pearl millet as it is a semi arid crop. The positive controls in both the GUS and MnSOD blots showed signals corresponding with the signals in the positive sample lanes. This result was consistent with the PCR results.

**TABLE 1**

| CALLI STAGE | NO OF CALLI/ PLATE | OSM | DIST | %TRANS |
|---|---|---|---|---|
| MS5 | 50 | 4hrs | 3cms | NIL |
| MS5 | 50 | 4hrs | 9cms | NIL |
| MS5 | 50 | 4hrs | 6cms | 1% |
| MS3 | 50 | 4hrs | 12cms | 5% |
| MS3 | 50 | 4hrs | 9cms | 11% |
| MS3 | 50 | 4hrs | 6cms | 89% |
| MS3 | 50 | 4hrs | 3cms | NIL |
| MS1 | 50 | 4hrs | 3cms | 1% |
| MS1 | 50 | 4hrs | 6cms | NIL |

| | | | | |
|---|---|---|---|---|
| OSM - the time period of osmotic treatment for the calli DIST - the distance of the calli from the stopping screen TRAN- the transformation efficiency | | | | |

Table 1: Shows the transformation efficiency of pearl millet 843B.
GUS assay revealed varied results for different stages of calli. MS3 stage calli showed the best transformation efficiency at 6cms distance.

### References

1. Pascal Lambe, Monique Dinant, Rene.F.Matagne, Differential long term expression and methylation of the hph and GUS genes in transgenic pearl millet callus. Plant science 108 (1995) 51-62
2. Vasil.,V and Vasil.,I.K, Somatic embryogenesis and plant regeneration from tissue cultures of P.americanum x P.purpureum hybrid Amer.J.Bot, 68(6)-864-872,1981
3. Hagio.,Takashi, optimizing the particle bombardment method for efficient genetic transformation, JARQ 32,239-247(1998)
4. Vasil.,v,Vasil.,I.K, somatic embryogenesis and plant regeneration from the suspension cultures of pearl millet, Amer. J. Bot, 47,(1981) 669-698.
5. Hunold, Bronner, Hahne.,G, early events in microprojectile bombardment:cell viability and particle location, The Plant Journal (1994),5(4), 593-604
6. Kohli.,A, Gahakwa.,D, Philippe vain, Paul Christou,Transgene expression in rice engineered through particle bombardment,Planta(1999) 208 ;88-97
7. Michael Nuccio, Rhodes,Hanson.,A.D, Metabolic engineering of plants for osmotic stress resistance ,Plant biotechnology, (2000), 128-134
8. Edwards.,K, Johnstone, Thompson, .C, A simple and rapid method for the preparation of plant genomic DNA for PCR analysis, N.A.Research, (1991)19, No.6 1349
9. Alan.,H , Peter.H, Ubiquitine Promoter based vectors for high level expression and screenable markers genes in monocotyledonous plants ,Transgenic research 5,213-218 (1996)
10. Philippe Vain, McMullen.,Michael, J.,John,Osmotic treatment enhances particle bombardment mediated transient and stable transformation of maize, Plant cell Reports(1993) 12;84-88

## Claims

1. A process for biolistic transformation and regeneration of *Pennisetum glaucum* (Pearl millet) comprising:
(a) initiating embryogenic calli formation from the seeds of *P. glaucam* in an MS media containing 5mg/L of 2,4 D
(b) incubating the said calli in dark for a predetermined period
(c) sub-culturing the calli on an MS media containing 3mg/L of 2,4 D,
(d) incubating said sub cultured calli under light for a predetermined period
(e) subjecting the embryogenic calli to biolistic bombardment with plasmid DNA containing pre-identified genes using a biolistic apparatus,
(f) allowing the proliferating calli to grow and differentiate into plantlets
(g) analysing the expression of said pre-identified genes in the regenerated plantlets using known techniques.

2. A process as claimed in claim 1 wherein the variety of *P. glaucam* used is *P.glaucam var. 843B.*

3. A process as claimed in claim 2 wherein the pre-identified gene is a marker gene selected from GUS encoding gene, Hygromycin resistance gene or MnSOD gene.

4. A process as claimed in claim 1 wherein the plasmids used in step (e) for transforming genes are selected from pIG121Hm and pGV4 construct.

5. A process as claimed in claim 4 said pIG 121 Hm plasmid is used for conferring GUS and hygromycin resistance gene and contains CaMV35s promoter.

6. A process as claimed in claim 4 wherein pGV4 plasmid is used in step (e) is for providing MnSOD gene and contains ubiquitin promoter and NOS terminator.

7. A process as claimed in claim 1 wherein the media used for differentiating the said calli obtained in step (f) into shoots, is MS basal medium with 0.3ppm of Benzyl Amino purine (BAP).

8. A process as claimed in claim 7 wherein the said calli was kept under light for a period of about 30 days for shoot formation.

9. A process as claimed in claim 1 wherein the media used for differentiating the said calli obtained in step (f) into root, is MS media with 2-3 ppm, Indole Acetic Acid (IAA) or 0.3 % of activated charcoal.

10. A process as claimed in claim 1 further comprising acclimatizing the plant in sterile water for at least 2 days and transferring it into sterile soil.

11. A process as claimed in claim 10 wherein said sterile soil contains soil and sand in the ratio 1:1.

12. A process as claimed in claim 1 wherein the calli is incubated under dark conditions in step (b) for 30 days.

13. A process as claimed in claim 1 wherein the calli is incubated under light conditions in step (d) for 30 days.

14. A process as claimed in claim 1 wherein said biolistic gun used for transformation is the PDS-1000/He gun.

15. A process as claimed in claims 1 or 9 wherein the biolistic gun uses high velocity microcarriers selected from gold.

16. A process as claimed in claim 15 wherein the said microcarriers are coated with DNA molecule, spermidine and CaCl₂.

17. A process as claimed in claim 15 wherein concentration of said DNA molecule is about 5 µg/µl, 0.1M spermidine and 2.5 M CaCl₂.

18. A process as claimed in claim 15 wherein the size of gold used is between 1.5 - 3.0 µ.

19. A process as claimed in claim 14 wherein the rupture disc pressure was 900 psi, the helium pressure is 1100psi and a vacuum of 25mg/Hg was created in the gun chamber.

20. A process as claimed in claims 1 and 14 to 19 wherein the calli was bombarded at step (c) at 6cms distance under rupture disc pressure of 900 psi and helium pressure of 1100psi.
